# EUROPÄISCHE PATENTSCHRIFT

(11) **EP 1 078 913 B1**
(45) Veröffentlichungstag und Bekanntmachung des Hinweises auf die Patenterteilung: **24.03.2004**
(21) Anmeldenummer: 00117576.9
(22) Anmeldetag: 16.08.2000
(51) Int. Cl.: C07C 69/54, C07C 67/03, C07C 67/62

(54) **Verfahren zur Herstellung von Di(meth)acrylsäureestern**
Process for the preparation of diesters of (meth)acrylic acid
Procédé de préparation de diesters de l'acide (méth)acrylique

(30) Priorität: 27.08.1999 DE 19940622
(43) Veröffentlichungstag der Anmeldung: 28.02.2001
(73) Patentinhaber: Röhm GmbH & Co. KG, 64293 Darmstadt (DE)
(72) Erfinder: Knebel, Joachim, Dr., 64665 Alsbach-Hähnlein (DE); Carl, Joachim, Dr., 64342 Seeheim-Jugenheim (DE); Gräff, Günther, 64665 Alsbach-Hähnlein (DE); Wittkowski, Andrea, 64823 Gross-Umstadt (DE)

(56) Entgegenhaltungen:
- EP-A- 0 236 994
- DE-A- 2 805 702
- US-A- 5 856 611

## Beschreibung

Die vorliegende Erfindung betrifft Verfahren zur Herstellung von Di(meth)acrylsäureestern durch Umesterung von (Meth)acrylsäureestern von C₁-C₄-Alkoholen mit 1,n-Diolen, worin n ≥ 3 ist, in Gegenwart von Metallverbindungen als Katalysatoren, wobei man als Metallverbindung Chelate des Zirconiums mit 1,3-Dicarbonylverbindungen einsetzt.

Di(meth)acrylsäureester werden im allgemeinen durch die katalysierte Umesterung von (Meth)acrylsäureestern erhalten. Katalysatoren für diese Umesterung sind in der Fachwelt weithin bekannt. So werden alkalische Katalysatoren, beispielsweise Lithium- und Calciumhydroxide verwendet, wie diese beispielsweise in den Offenlegungsschriften DE-OS 34 23 441 und 34 23 443, beschrieben sind. Bei Einsatz dieser basischen Katalysatoren muß allerdings mit Nebenreaktionen gerechnet werden, wie beispielsweise der Michael-Addition, die sowohl die Reinheit der gewünschten Diester als auch deren Ausbeute schmälert.

Des weiteren sind Zirconiumkomplexe bekannt, die zur Katalyse der Reaktion zwischen den Estern und den Alkoholen verwendet werden können. Diese Komplexe führen zu sehr hohen Umsätzen und einer hohen Reinheit der Produkte, da diese Katalysatoren neutral sind. Ein weiterer Vorteil dieser Katalysatoren besteht darin, daß die Alkohole vor der Umesterung nicht getrocknet werden müssen. Diese Katalysatoren sind beispielsweise in der deutschen Offenlegungsschrift DE-OS 28 05 702 und in der europäischen Patentschrift EP 0 236 994 B1 beschrieben.

Darüber hinaus beschreibt FR-A-2 747 675 ein Verfahren zur Umesterung von (Meth)acrylaten, bei dem Zirconiumkatalysatoren in situ gebildet werden.

Die Reaktivität dieser Katalysatoren ist sehr hoch'. Nach der Umsetzung müssen sie allerdings möglichst vollständig abgetrennt werden, da die erhaltenen Mischungen trüb sind und die Katalysatoren unvorhergesehene Wirkungen auf nachfolgende Reaktionen beim Anwender haben könnten. Vor einem Verkauf der Di(meth)acrylsäureester müssen daher die zirconiumenthaltenden Katalysatoren abgetrennt werden.

Diese Abtrennung ist jedoch äußerst aufwendig. So wurde der Katalysator bisher nach einer Hydrolyse durch Zentrifugation abgetrennt, wonach häufig noch destilliert werden mußte, um Produkte ohne Trübungen zu erhalten, die die für viele Anwendungszwecke erforderliche Reinheit aufweisen.

Dementsprechend ist es Aufgabe der vorliegenden Erfindung Verfahren zur Herstellung von Di(meth)acrylsäureestern durch Umesterung von (Meth)acrylsäureestern von C₁-C₄-Alkoholen mit 1,n-Diolen, worin n ≥ 3 ist, in Gegenwart von Metallverbindungen als Katalysatoren zur Verfügung zu stellen, wobei man als Metallverbindung Chelate des Zirconiums mit 1,3-Dicarbonylverbindungen einsetzt, durch die die gewünschten Di(meth)acrylsäureester in hoher Reinheit besonders kostengünstig erhalten werden.

Des weiteren lag der Erfindung die Aufgabe zugrunde ein Verfahren bereitzustellen, bei dem der Katalysator ohne energieintensive Destillation von dem Produkt abgetrennt werden kann.

Gelöst werden diese Aufgaben sowie weitere, die zwar nicht wörtlich genannt werden, die sich aber aus den hierin diskutierten Zusammenhängen, wie selbstverständlich ableiten lassen oder sich'aus diesen zwangsläufig ergeben, durch Verfahren zur Herstellung von Di(meth)acrylsäureestern mit allen Merkmalen des unabhängigen Anspruchs 1.

Vorteilhafte Ausgestaltungen des erfindungsgemäßen Verfahrens sind Gegenstand der auf die unabhängigen Verfahrensansprüche rückbezogenen Ansprüche.

Dadurch, daß man den Katalysator nach der Umesterung mit Phosphorsäure ausfällt und den entstandenen Niederschlag abtrennt, gelingt es ein Verfahren zur Herstellung von Di(meth)acrylsäureestern durch Umesterung von (Meth)acrylsäureestern von C₁-C₄-Alkoholen mit 1,n-Diolen, worin n ≥ 3 ist, in Gegenwart von Metallverbindungen als Katalysatoren, wobei man als Metallverbindung Chelate des Zirconiums mit 1,3-Dicarbonylverbindungen einsetzt, zur Verfügung zu stellen, durch welches die gewünschten Di(meth)acrylsäureester in hoher Reinheit, insbesondere ohne störende Zirconiumverbindungen, besonders kostengünstig erhalten werden.

Durch die erfindungsgemäßen Maßnahmen werden unter anderem insbesondere folgende Vorteile erzielt:
⇒ Erfindungsgemäße Verfahren führen zu sehr hohen Umsätzen und einer hohen Reinheit der Produkte.
⇒ Die eingesetzten 1,n-Diole, wobei n ≥ 3 ist, müssen nicht vor ihrer Verwendung getrocknet werden.
⇒ In dem Verfahren können preisgünstige Zirconiumverbindungen eingesetzt werden, da der Katalysator in situ durch Zugabe von 1,3-Dicarbonylverbindungen hergestellt werden kann.
⇒ Nach der Abtrennung der durch Phosphorsäure gefällten Zirconiumverbindungen muß das Endprodukt nicht mehr destillativ gereinigt werden.
⇒ Durch das erfindungsgemäße Verfahren werden Produkte erhalten, die keine Trübung aufweisen.

Die Schreibweise Di(meth)acrylsäureester umfaßt Diester der Methacrylsäure, der Acrylsäure sowie Mischungen aus beiden Säuren.

Im Rahmen der vorliegenden Erfindung herstellbare Di(meth)acrylsäureester lassen sich im allgemeinen durch die Formel 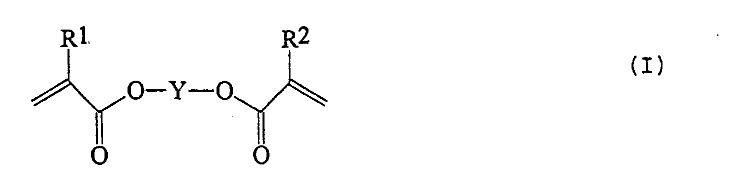 darstellen, worin
die Reste R¹ und R² gleich oder verschieden Wasserstoff oder eine Methylgruppe darstellen,
der Rest Y eine divalente Verbindungsgruppe darstellt, wobei die beiden (Meth)acrylsäurereste durch mindestens 3 Kohlenstoffatome getrennt sind. Diese Verbindungsgruppen leiten sich von den 1,n-Diolen ab, die bei der Umesterung eingesetzt werden.

Beispiele für besonders bevorzugte Verbindungsgruppe Y sind geradkettige, verzweigte oder cyclische Alkylreste, die sowohl gesättigt als auch ungesättigt sein können, wie beispielsweise Propyl, Butyl, Pentyl, Hexyl, Cyclopentyl, Cyclohexyl, Pentenyl, sowie Polyetherglykole. Verbindungsgruppen können reaktive Gruppen aufweisen. Zu diesen reaktiven Gruppen gehören beispielsweise halogenhaltige Gruppen, Epoxygruppen, aromatische und heteroaromatische Gruppen sowie Thiolgruppen.

Im Rahmen der Erfindung einsetzbare (Meth)acrylsäureester von C₁-C₄ Alkoholen können durch die Formel 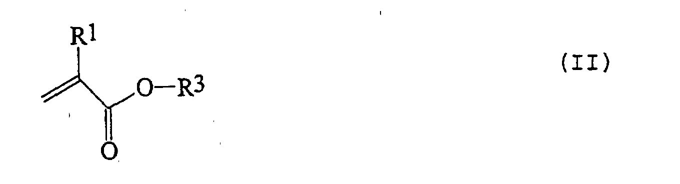 dargestellt werden, worin
der Rest R¹ Wasserstoff oder eine Methylgruppe und der Rest R³ eine Alkylgruppe mit 1 bis 4 Kohlenstoffatomen ist.

Alkylgruppen mit 1 bis 4 Kohlenstoffatomen sind beispielsweise Methyl, Ethyl, n-Propyl, iso-Propyl, n-Butyl, iso-Butyl und tert-Butyl. Diese Gruppe können sowohl unsubstituiert als auch substituiert sein.

Bei der Umesterung können die Acrylsäure- und/oder Methacrylsäureester sowohl allein als auch als Mischung eingesetzt werden.

Die oben genannten (Meth)acrylsäureester sind im allgemeinen kommerziell erhältlich, wobei Methylacrylat und Methylmethacrylat besonders bevorzugt sind. Diese Substanzen sind besonders kostengünstig. Des weiteren läßt sich bei der Umesterung freiwerdendes Methanol leicht durch Destillation aus dem Reaktionsgemisch entfernen, so daß sehr hohe Umsätze erzielt werden können.

1,n-Diole, wobei n ≥ 3 ist, umfassen insbesondere Verbindungen der Formel

HO-Y-OH (III),

worin Y die gleiche Bedeutung wie in Formel I hat.

Zu den bevorzugten 1,n-Diolen gehören unter anderem Alkoxyalkandiole, Alkenoxyalkandiole, Alkendiole, Glycole, Polyetherglycole, Phenoxyalkandiole, Alkylphenoxyalkandiole, Phenylalkandiole, Alkylphenylalkandiole, Alkylmorpholinoalkandiole, Alkylpiperidinoalkandiole, Pyridylalkandiole, Halogenalkandiole.

Besonders bevorzugte Beispiele für 1,n-Diole sind 1,3-Propandiol, n-Butan-1,3-diol, 2-Methyl-1,3-Propandiol, Neopentylglycol (2,2-Dimethyl-1,3-Propandiol), 1,4-Butandiol, Triethylenglycol und Polyethylenglycol-400.

Ganz besonders bevorzugte 1,n-Diole zeichnen sich dadurch aus, daß n = 3, 4 oder 6 ist.

Die 1,n-Diole können allein oder in Form von Mischung eingesetzt werden. Sie sind im allgemeinen kommerziell erhältlich, wobei ihre Herstellung in der Fachwelt weithin bekannt ist.

Die als Katalysatoren in der Umsetzung eingesetzten Chelatkomplexverbindungen des Zirconiums mit 1,3-Dicarbonylverbindungen sind dem Fachmann wohlbekannt.

Zu den im Rahmen der Erfindung mit besonderem Erfolg einsetzbaren 1,3-Dicarbonylverbindungen gehören unter anderem Acetessigester, Acetylacetonat, 2,4-Hexandionat, 3,5-Heptandionat, 3-Methylacetylacetonat, 3-Phenylacetylacetonat, 4,4,4-Trifluor-1-phenyl-1,3-butandionat, 2,2,6,6-Tetramethyl-3,5-heptandionat, 1,1,1-Trifluor-5,5-dimethyl-2,4-hexandionat, 1,1,1-Trifluor-2,4-pentandionat und Dibenzoylmethan. Besonders bevorzugt ist Acetylaceton.

Die Herstellung der Zirconiumchelate aus 1,3-Dicarbonylen und Zirconiumverbindungen sowie ihre Verwendung ist beispielsweise in Houben-Weyl, Methoden der organischen Chemie, 4. Auflage, Bd. VI/2, 1963, Seiten 53-55 und 58 bis 61 sowie in A.E. Martell, M. Calvin, "Die Chemie der Metallchelatverbindungen" (1958) beschrieben.

Es wurde von den Erfindern der vorliegenden Erfindung überraschend festgestellt, daß katalytisch wirksame Zirconiumkomplexe auch in situ durch Zugabe von preisgünstigen Zirconiumverbindungen, wie beispielsweise Tetrabutoxyzirconium, und den oben beschriebenen 1,3-Diketonen, insbesondere Acetylacetonat, zu den Reaktanten hergestellt werden kann.

Die im Rahmen der vorliegenden Erfindung ganz besonders bevorzugt als Katalysator einsetzbare Verbindung ist Zirconiumacetylacetonat.

Bei dem erfindungsgemäßen Verfahren können die Zirconium-Katalysatoren einzeln oder als Mischung eingesetzt werden. Die Mengen, die zur Umesterung eingesetzt werden, liegen im Bereich von 0,01 bis 10 Mol%, vorzugsweise von 0,05 bis 10 Mol%, bezogen auf die Mole an 1,n-Diolen.

Die (Meth)acrylsäureester der Formel (II) werden besonders vorteilhaft in Mengen im Bereich 2 bis 20 Mol, insbesondere im Bereich von 3 bis 12 Mol auf 1 Mol 1,n-Diol eingesetzt.

Um eine unerwünschte Polymerisation der (Meth)acrylate zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tertbutylbrenzcatechin, Phenothiazin, p-Phenylendiamin, Methylenblau oder sterisch gehinderte Phenole, sind in der Fachwelt weithin bekannt. Diese Verbindungen können einzeln oder in Form von Mischungen eingesetzt werden und sind im allgemeinen kommerziell erhältlich.

Bezogen auf das Gewicht der gesamten Reationsmischung kann der Anteil der Inhibitoren einzeln oder als Mischung im allgemeinen 0,01 - 0,5 % (wt/wt) betragen.

Zur Inhibierung kann aber auch Sauerstoff eingesetzt werden. Hierbei kann dieser auch in Form von Luft verwendet werden, wobei die Mengen vorteilhaft so dosiert werden, daß der Gehalt in der Gasphase über dem Reaktionsgemisch unterhalb der Explosionsgrenze bleibt. Besonders bevorzugt sind hierbei Mengen im Bereich von 0,1 bis 1 1 pro Stunde und Mol 1,n-Diol.

Die Umesterung kann unter Normal-, Unter- sowie Überdruck stattfinden. Die Reaktionstemperaturen können in einem weiten Bereich gewählt werden, die im allgemeinen vom eingesetzten Druck abhängig sind. Vorteilhafte Temperaturen liegen beispielsweise im Bereich von 30 bis 150 °C, insbesondere von 50 bis 130 °C und ganz besonders bevorzugt von 70 bis 120°C.

Die Umesterung kann sowohl kontinuierlich als auch chargenweise durchgeführt werden. Das erfindungsgemäße Verfahren kann in Substanz, d.h. ohne Verwendung eines weiteren Lösungsmittels durchgeführt werden. Falls gewünscht kann auch ein inertes Lösungsmittel eingesetzt werden. Hierzu gehören unter anderem Benzol, Toluol, n-Hexan, Cyclohexan und Methylisobutylketon (MIBK), Methylethylketon (MEK).

Bei einer besonders zweckmäßigen Varianten der erfindungsgemäßen Umesterung werden sämtliche Komponenten, wie beispielsweise das 1,n-Diol, der (Meth)acrylsäureester sowie der Katalysator, gemischt, wonach diese Reaktionsmischung bis zum Sieden erwärmt wird. Hierbei wird zunächst Wasser, das in dem Alkohol enthalten sein kann, azeotrop mit (Meth)acrylsäure abgetrennt. Anschließend wird der frei werdende C₁-C₄-Alkohol destillativ, möglicherweise azeotrop mit (Meth)acrylsäure, aus der Reaktionsmischung entfernt.

Die Reaktionszeiten sind beispielsweise von den gewählten Parametern, wie Druck und Temperatur, den Reaktanten, wie (Meth)acrylsäure und 1,n-Diol, sowie dem Katalysator abhängig. Sie liegen aber im allgemeinen im Bereich von 1 bis 12 Stunden, bevorzugt von 3 bis 8 Stunden. Weitere Hinweise, in bezug auf die Reaktionszeiten kann der Fachmann den beigefügten Beispielen entnehmen.

Gemäß der vorliegenden Erfindung wird der Zirconiumkatalysator nach der Umsetzung aus der Mischung durch Zugabe von Phosphorsäure abgetrennt. Die Phosphorsäure kann der Reaktionsmischung in reiner Form sowie als Lösung beigemischt werden. Besonders vorteilhaft wird die Phosphorsäure als wäßrige Lösung zugegeben. Die Konzentration der wäßrigen Lösung kann beispielsweise im Bereich von 0,5 bis 90 Gew.-% liegen. Die Konzentration hängt insbesondere auch von der verwendeten 1,n-Diolverbindung ab, wie dies den Beispielen entnommen werden kann. Die Menge der eingesetzten Phosphorsäure richtet sich sowohl nach der eingesetzten Zirconiumverbindung als auch nach dem verwendeten 1,n-Diol. Im allgemeinen liegt sie im Bereich von 0,5 bis 2 Mol, pro Mol an zu fällender Zirconiumverbindung.

Durch die Zugabe von Phosphorsäure bildet sich ein Niederschlag, der auf jede dem Fachmann bekannte Weise von der Reaktionsmischung getrennt werden kann. Hierzu gehören unter anderem Zentrifugieren, Dekantieren, Destillieren und Filtrieren. Aufgrund der Wirtschaftlichkeit ist das Filtrieren besonders bevorzugt.

Nach dem Abtrennen des Niederschlags weisen die nach der erfindungsgemäßen Umesterung erhaltenen Di(meth)acrylate ohne destillative Aufreinigung im allgemeinen einen Zirconiumgehalt von weniger als 1,5 ppm, bevorzugt weniger als 0,7 ppm und ganz besonders bevorzugt weniger als 0,1 ppm, gemessen als Metall, auf.

Je nach Anwendungszweck können die so erhaltenen Di(meth)acrylsäureester ohne weitere Aufreinigung eingesetzt werden. Geringe Mengen an verbliebenem 1,n-Diol stören im allgemeinen die nachfolgenden Polymerisationsreaktionen nicht, wobei Umsätze erzielt werden, die in der Regel wesentlich größer als 90% sind. Die Di(meth)acrylsäureester werden häufig zusammen mit den acrylischen Ausgangssubstanzen verwendet, so daß Reste dieser Reaktanten nicht abgetrennt werden müssen.

Hierbei sind die erhaltenen Reaktionsmischungen nach der Ausfällung des Katalysators durch die Phosphorsäure klar.

Für besondere Einsatzzwecke können die durch das vorliegenden Verfahren erhaltenen Produkte aber auch auf jede in der Fachwelt weithin bekannte Methode aufgereinigt werden.

Die nachfolgenden Beispiele und Vergleichsbeispiele dienen zur detaillierten Beschreibung der vorliegenden Erfindung, ohne daß hierdurch eine Einschränkung erfolgen soll. Die Angaben in Prozent beziehen sich auf das Gesamtgewicht, es sei denn anderes ist vermerkt.

### Beispiel 1

In eine Apparatur, die einen 11-Dreihalskolben mit mechanischem Rührer, Lufteinleitung und aufgesetzter Füllkörperkolonne (30 cm lang, gefüllt mit 6 mm Raschigringen) sowie Kolonnenkopf mit Rücklaufteiler aufwies, wurden 67,5 g 1,3-Propandiol und 532 g Methylmethacrylat (MMA) gegeben. Um eine unerwünschte Polymerisation zu verhindern, wurden 500 ppm Hydrochinonmonomethylether hinzugefügt. Unter Lufteinleitung wurde die Reaktionsmischung zum Sieden erhitzt. Entstehendes MMA-Wasser-Azeotrop, das sich bei Verwendung von feuchten Einsatzstoffen bildete, wurde solange abdestilliert, bis die Kopftemperatur konstant blieb (ca. 100 °C) und klares MMA überging.

Die Reaktionsmischung wurde auf ca. 90 °C abgekühlt, das als Azeotrop abdestillierte MMA wurde durch frisches ersetzt und 6 g (1% bezogen auf die Reaktanten) Zirconiumacetylacetonat wurde der Mischung beigefügt. Anschließend wurde die Reaktionsmischung erneut zum Sieden erhitzt, wobei das gebildete MMA-Methanol-Azeotrop destillativ aus der Reaktionsmischung entfernt wurde. Die Kopftemperatur steigt hierbei von 65 auf ca. 99 °C an, wobei schließlich der Siedepunkt von reinem MMA erreicht wurde. Nach 6 Stunden war die Reaktion beendet. Zu diesem Zeitpunkt ging nur noch reines MMA über. Die Reaktionsmischung wurde abgekühlt. Anschließend wurden der Mischung 25 ml 10%iger Phosphorsäure (wäßrige Lösung) zugegeben, wodurch der Zirconiumkatalysator ausfiel. Der gebildete Niederschlag wurde durch Filtrieren abgetrennt und überschüssiges MMA wurde im Vakuum aus der Mischung entfernt.

Die Mischung wurde nachfolgend durch Gaschromatographie analysiert. Die Ausbeute betrug 140 g. Das Endprodukt enthielt gemäß GC-Analyse
94,2 % 1,3-Propandioldimethacrylat,
5,2 % 1,3-Propandiolmonomethacrylat und
0,2 % 1,3-Propandiol.

### Beispiel 2

Das obige Beispiel 1 wurde im wesentlichen wiederholt, wobei jedoch 78,4 g 1,3-Butandiol und 522 g MMA als Reaktanten eingesetzt wurden. Diese Mischung wurde mit 200 ppm Hydrochinonmonomethylether stabilisiert. Die Umsetzung dauerte 8 Stunden.

Nach Beendigung der Umesterung wurde der Katalysator mit 19 ml 1%iger Phosphorsäure gefällt. Die Ausbeute betrug 157 g. Gemäß GC-Analyse enthielt das Endprodukt
98,6 % 1,3-Butandioldimethacrylat und
0,17 % 1,3-Butandiolmonomethacrylat. Der Zirconiumgehalt wurde durch Atomabsorptionsspektroskopie (AAS) bestimmt,
wobei dieser kleiner als 0,1 ppm war.

### Beispiel 3

Das obige Beispiel 1 wurde im wesentlichen wiederholt,
wobei jedoch 78,3 g 2-Methyl-1,3-propandiol und 522 g MMA als Reaktanten eingesetzt wurden. Diese Mischung wurde mit 200 ppm Hydrochinonmonomethylether stabilisiert. Die Umsetzung dauerte 8 Stunden.

Nach Beendigung der Umesterung wurde der Katalysator mit 25 ml 10%iger Phosphorsäure gefällt. Die Ausbeute betrug 175 g. Gemäß GC-Analyse enthielt das Endprodukt
98,6 % 2-Methyl-1,3-propandioldimethacrylat und
0,08 % MMA.

### Beispiel 4

Das obige Beispiel 1 wurde im wesentlichen wiederholt,
wobei jedoch 88,4 g Neopentylglycol und 510 g MMA als Reaktanten eingesetzt wurden. Diese Mischung wurde mit 200 ppm Hydrochinonmonomethylether stabilisiert. Die Umsetzung dauerte 5 Stunden.

Nach Beendigung der Umesterung wurde der Katalysator mit 25 ml 10%iger Phosphorsäure gefällt. Die Ausbeute betrug 190 g. Gemäß GC-Analyse enthielt das Endprodukt 97,5 % Neopentylglycoldimethacrylat und 1,2 % MMA.

### Beispiel 5

Das obige Beispiel 1 wurde im wesentlichen wiederholt,
wobei jedoch 78,4 g 1,4-Butandiol und 522 g MMA als Reaktanten eingesetzt wurden. Diese Mischung wurde mit 200 ppm Hydrochinonmonomethylether stabilisiert. Die Umsetzung dauerte 4 Stunden.

Nach Beendigung der Umesterung wurde der Katalysator mit 25 ml 10%iger Phosphorsäure gefällt. Die Ausbeute betrug 177 g. Gemäß GC-Analyse enthielt das Endprodukt
99 % 1,4-Butandioldimethacrylat,
0,14 % 1,4-Butandiolmonomethacrylat, 0,09 % 1,4-Butandiol und 0,23 % MMA. Der Zirconiumgehalt gemäß AAS war kleiner als 0,1 ppm.

### Beispiel 6

Das obige Beispiel 1 wurde im wesentlichen wiederholt,
wobei jedoch 560 g Polyethylenglycol-200 und 1500 g MMA als Reaktanten eingesetzt wurden, welche in einen 41-Dreihalskolben gegeben wurden. Diese Mischung wurde mit 250 ppm Hydrochinonmonomethylether stabilisiert. Die eingesetzte Menge an Zirconiumacetylacetonat betrug 25 g (1 % bezogen auf die Reaktanten). Die Umsetzung dauerte 7 Stunden.

Nach Beendigung der Umesterung wurde der Katalysator mit 100 ml 10%iger Phosphorsäure gefällt. Die Ausbeute betrug 860 g. Gemäß GC-Analyse enthielt das Endprodukt
96,8 % Polyethylenglycol-200-dimethacrylat,
1 % Polyethylenglycol-200-monomethacrylat und 0,8 % MMA.

### Beispiel 7

Das obige Beispiel 1 wurde im wesentlichen wiederholt,
wobei jedoch 70 g Polyethylenglycol-200 und 238 g MMA als Reaktanten eingesetzt wurden. Die eingesetzte Menge an Zirconiumacetylacetonat betrug 3 g (0,5 % bezogen auf die Reaktanten). Diese Mischung wurde mit 250 ppm Hydrochinonmonomethylether stabilisiert. Bei dieser Umesterung wurde auf eine Entwässerung der Reaktionsmischung verzichtet. Die Umsetzung dauerte 6 Stunden.

Nach Beendigung der Umesterung wurde der Katalysator mit 12,5 ml 10%iger Phosphorsäure gefällt. Die Ausbeute betrug 107 g. Gemäß GC-Analyse enthielt das Endprodukt
96,7 % Polyethylenglycol-200-dimethacrylat, 0,6 % Polyethylenglycol-200-monomethacrylat und 1,3 % MMA.

### Beispiel 8

Das obige Beispiel 1 wurde im wesentlichen wiederholt,
wobei jedoch 600 g Triethylenglycol und 1800 g MMA als Reaktanten eingesetzt wurden, welche in einen 41-Dreihalskolben gegeben wurden. Diese Mischung wurde mit 1000 ppm Hydrochinonmonomethylether und 250 ppm 2,4-Bis(tert.-butyl)-p-kresol stabilisiert. Die eingesetzte Menge an Zirconiumacetylacetonat betrug 24 g (1 % bezogen auf die Reaktanten). Die Umsetzung dauerte 6 Stunden.

Nach Beendigung der Umesterung wurde der Katalysator mit 97 ml 10%iger Phosphorsäure gefällt. Die Ausbeute betrug 1070 g. Gemäß GC-Analyse enthielt das Endprodukt
96,5 % Triethylenglycoldimethacrylat, 1,12 % Triethylenglycolmonomethacrylat und 1 % MMA. Der Zirconiumgehalt gemäß AAS betrug 0,6 ppm.

### Beispiel 9

Das obige Beispiel 1 wurde im wesentlichen wiederholt,
wobei jedoch 100 g Polyethylenglycol-400 und 200 g MMA als Reaktanten eingesetzt wurden, welche in einen 500 ml Dreihalskolben gegeben wurden. Diese Mischung wurde mit 500 ppm Hydrochinonmonomethylether und 250 ppm 2,4-Bis(tert.-butyl)-p-kresol stabilisiert. Die eingesetzte Menge an Zirconiumacetylacetonat betrug 3 g (1 % bezogen auf die Reaktanten). Die Umsetzung dauerte 3,5 Stunden.

Nach Beendigung der Umesterung wurde der Katalysator mit 12,5 ml 10%iger Phosphorsäure gefällt. Die Ausbeute betrug 119 g. Gemäß GC-Analyse enthielt das Endprodukt
96,7 % Polyethylenglycol-400-dimethacrylat und 0,07 % Polyethylenglycol-400-monomethacrylat. Der Zirconiumgehalt gemäß AAS betrug 1,3 ppm.

### Beispiel 10

Das obige Beispiel 1 wurde im wesentlichen wiederholt,
wobei jedoch 78,4 g 1,4-Butandiol und 522 g MMA als Reaktanten eingesetzt wurden. Diese Mischung wurde mit 120 ppm Hydrochinonmonomethylether und 3 mg 2,2,6,6-Tetramethylpiperidyl-N-oxyl stabilisiert. Zu dieser Zusammensetzung wurden 2,9 g (6,15 mmol) einer 70%-igen Lösung von Tetra-n-propylzirconat in n-Propanol gegeben, wodurch ein Niederschlag gebildet wurde. Nach Zugabe von 2,5 g (25 mmol) frisch destilliertes Acetylaceton löste sich der zuvor gebildete Niederschlag auf, wonach die Zusammensetzung auf Siedetemperatur erhitzt wurde. Die Umsetzung dauerte 2 Stunden. Anschließend wurden leicht flüchtige Verbindungen, wie überschüssiges MMA, im Vakuum (1 mbar) abgetrennt.

Nach Beendigung der Umesterung wurde der Katalysator mit 0,83 ml 85%iger Phosphorsäure bei Raumtemperatur gefällt. Die Ausbeute betrug 185 g. Gemäß GC-Analyse enthielt das
Endprodukt 98,6 % 1,4-Butandioldimethacrylat,
0,032 % 1,4-Butandiolmonomethacrylat, 0,6 % Acetylaceton und 0,015% MMA.

### Vergleichsbeispiel 1

Das obige Beispiel 1 wurde im wesentlichen wiederholt, wobei jedoch 62 g Ethylenglycol und 600 g MMA als Reaktanten eingesetzt wurden. Die eingesetzte Menge an Zirconiumacetylacetonat betrug 6,62 g (1 % bezogen auf die Reaktanten). Diese Mischung wurde mit 70 ppm Hydrochinonmonomethylether, 40 ppm N,N'-Diphenylp-phenylendiamin und 10 ppm 2,2,6,6-Tetramethyl-4-hydroxypiperidin-N-oxyl stabilisiert. Nach 5,5 Stunden wurde die Umsetzung abgebrochen, da kein Methanol gebildet wurde.

### Vergleichsbeispiel 2

Das obige Beispiel 1 wurde im wesentlichen wiederholt,
wobei jedoch 140 g (0,7 mol) Polyethylenglycol-200 und 477 g (4,77 mol) MMA als Reaktanten eingesetzt wurden. Die eingesetzte Menge an Zirconiumacetylacetonat betrug 6,2 g (1 % bezogen auf die Reaktanten). Diese Mischung wurde mit 0,059 g (250 ppm bezogen auf die Reaktanten) Hydrochinonmonomethylether und 5,9 mg (25 ppm bezogen auf die Reaktanten) 2,2,6,6-Tetramethyl-4-hydroxy-piperidin-N-oxyl stabilisiert. Bei dieser Umesterung wurde auf eine Entwässerung der Reaktionsmischung verzichtet. Die Umsetzung dauerte 5,5 Stunden.

Nach Beendigung der Umesterung wurde der Katalysator mit 12,5 ml 10%iger Harnstofflösung bei Raumtemperatur gefällt, wonach der Niederschlag durch Filtration abgetrennt wurde. Die Ausbeute betrug 228 g. Gemäß GC-Analyse enthielt das Endprodukt 96,9 % Polyethylenglycol-200-dimethacrylat und 1,07 % Polyethylenglycol-200-monomethacrylat. Das Produkt weist einen Zirconiumgehalt von 43 ppm auf.

## Patentansprüche

1. Verfahren zur Herstellung von Di(meth)acrylsäureestern durch Umesterung von (Meth)acrylsäureestern von C₁-C₄-Alkoholen mit 1,n-Diolen, worin n ≥ 3 ist, in Gegenwart von Metallverbindungen als Katalysatoren, wobei man als Metallverbindung Chelate des Zirconiums mit 1,3-Dicarbonylverbindungen einsetzt,
**dadurch gekennzeichnet,**
**daß** man den Katalysator nach der Umesterung mit Phosphorsäure ausfällt und den entstandenen Niederschlag abtrennt.

2. Verfahren nach Anspruch 1,
**dadurch gekennzeichnet,**
**daß** man den Katalysator in situ aus mindestens einer Zirconiumverbindung und mindestens einer 1,3-Dicarbonylverbindung herstellt.

3. Verfahren nach Anspruch 2,
**dadurch gekennzeichnet,**
**daß** man als Zirconiumverbindung Tetrabutoxyzirconium und als 1,3-Dicarbonylverbindung Acetylacetonat einsetzt.

4. Verfahren nach einem oder mehreren der vorhergehenden Ansprüche,
**dadurch gekennzeichnet,**
**daß** man Di(meth)acrylsäureester erhält, die ohne destillative Aufreinigung einen Zirconiumgehalt von weniger als 1,5 ppm, gemessen als Metall, aufweisen.

5. Verfahren nach Anspruch 4,
**dadurch gekennzeichnet, ,**
**daß** man Di(meth)acrylsäureester erhält, die ohne destillative Aufreinigung einen Zirconiumgehalt von weniger als 0,7 ppm, gemessen als Metall, aufweisen.

6. Verfahren nach Anspruch 5,
**dadurch gekennzeichnet,**
**daß** man Di(meth)acrylsäureester erhält, die ohne destillative Aufreinigung einen Zirconiumgehalt von weniger als 0,1 ppm, gemessen als Metall, aufweisen.

7. Verfahren nach einem oder mehreren der vorhergehenden Ansprüche,
**dadurch gekennzeichnet,**
**daß** man einen oder mehrere Katalysatoren in einer Menge von 0,01 bis 10 Mol%, bezogen auf die Mole an 1,n-Diolen, einsetzt.

8. Verfahren nach einem oder mehreren der vorhergehenden Ansprüche,
**dadurch gekennzeichnet,**
**daß** man einen oder mehrere Katalysatoren in einer Menge von 0,05 bis 10 Mol%, bezogen auf die Mole an 1,n-Diolen, einsetzt.

9. Verfahren nach einem oder mehreren der vorhergehenden Ansprüche,
**dadurch gekennzeichnet,**
**daß** man die Umesterung in Gegenwart von Zirconiumacetylacetonat durchführt.

10. Verfahren nach einem oder mehreren der vorhergehenden Ansprüche,
**dadurch gekennzeichnet,**
**daß** man Methylmethacrylat oder Methylacrylat mit 1,n-Diolen, worin n = 3, 4 oder 6 ist, umsetzt.

11. Verfahren nach einem oder mehreren der vorhergehenden Ansprüche,
**dadurch gekennzeichnet,**
**daß** man 2 bis 20 Mol (Meth)acrylsäure auf 1 Mol 1,n-Diol einsetzt.

12. Verfahren nach einem oder mehreren der vorhergehenden Ansprüche,
**dadurch gekennzeichnet,**
**daß** man während der Reaktion das freiwerdende Alkanol des (Meth)acrylsäureesters kontinuierlich aus dem Reaktionsgemisch entfernt.

13. Verfahren nach einem oder mehreren der vorhergehenden Ansprüche,
**dadurch gekennzeichnet**,
man die Umsetzung in Gegenwart von Polymerisationsinhibitoren durchführt.

14. Verfahren nach einem oder mehreren der vorhergehenden Ansprüche,
**dadurch gekennzeichnet,**
**daß** man die Umsetzung in Gegenwart von Luftsauerstoff durchführt.

## Claims

1. Process for preparing di(meth)acrylic acid esters by transesterifying (meth)acrylic acid esters of C₁-C₄ alcohols with 1,n-diols wherein n ≥ 3, in the presence of metal compounds as catalysts, where chelates of zirconium with 1,3-dicarbonyl compounds are used as the metal compound, **characterised in that** after the esterification the catalyst is precipitated with phosphoric acid and the precipitate formed is separated off.

2. Process according to claim 1, **characterised in that** the catalyst is prepared *in situ* from at least one zirconium compound and at least one 1,3-dicarbonyl compound.

3. Process according to claim 2, **characterised in that** the zirconium compound is tetrabutoxy zirconium and the 1,3-dicarbonyl compound is acetyl acetonate.

4. Process according to one or more of the preceding claims, **characterised in that** di(meth)acrylic acid esters are obtained which, without purification by distillation, have a zirconium content of less than 1.5 ppm, measured as metal.

5. Process according to claim 4, **characterised in that** di(meth)acrylic acid esters are obtained which, without purification by distillation, have a zirconium content of less than 0.7 ppm, measured as metal.

6. Process according to claim 5, **characterised in that** di(meth)acrylic acid esters are obtained which, without purification by distillation, have a zirconium content of less than 0.1 ppm, measured as metal.

7. Process according to one or more of the preceding claims, **characterised in that** one or more catalysts are used in an amount of from 0.01 to 10 mol%, based on the moles of 1,n-diols.

8. Process according to one or more of the preceding claims, **characterised in that** one or more catalysts are used in an amount of from 0.05 to 10 mol%, based on the moles of 1,n-diols.

9. Process according to one or more of the preceding claims, **characterised in that** the transesterification is carried out in the presence of zirconium acetyl acetonate.

10. Process according to one or more of the preceding claims, **characterised in that** methyl methacrylate or methyl acrylate is reacted with 1,n-diols, wherein n = 3, 4 or 6.

11. Process according to one of more of the preceding claims, **characterised in that** 2 to 20 mol of (meth)acrylic acid are used to 1 mol of 1,n-diol.

12. Process according to one or more of the preceding claims, **characterised in that** during the reaction the alkanol of the (meth)acrylic acid ester liberated is continuously removed from the reaction mixture.

13. Process according to one or more of the preceding claims, **characterised in that** the reaction is carried out in the presence of polymerisation inhibitors.

14. Process according to one or more of the preceding claims, **characterised in that** the reaction is carried out in the presence of oxygen from the air.

## Revendications

1. Procédé de préparation d'esters de l'acide di(méth)acrylique par réaction d'esters d'acide (méth)acrylique d'alcools en C₁ à C₄ avec des 1,n-diols, où n ≥ 3, en présence de composés métalliques comme catalyseurs, en utilisant comme composé métallique des chélates de zirconium avec des composés 1,3-dicarbonyles,
**caractérisé en ce qu'**
on précipite le catalyseur après la trans-estérification avec l'acide phosphorique et on sépare le précipité apparu.

2. Procédé selon la revendication 1,
**caractérisé en ce qu'**
on prépare le catalyseur in situ à partir d'au moins un composé de zirconium et d'au moins un composé 1,3-dicarbonyle.

3. Procédé selon la revendication 2,
**caractérisé en ce qu'**
on utilise comme composé de zirconium le tétrabutoxy-zirconium, et comme composé 1,3-dicarbonyle on utilise l'acétyl acétonate.

4. Procédé selon une ou plusieurs des revendications précédentes,
**caractérisé en ce qu'**
on obtient des esters d'acide di(méth)acrylique qui présentent sans purification par distillation une teneur en zirconium inférieure à 1,5 ppm, mesurée sous forme de métal.

5. Procédé selon la revendication 4,
**caractérisé en ce qu'**
on obtient des esters d'acide di(méth)acrylique qui présentent sans purification par distillation une teneur en zirconium inférieure à 0,7 ppm, mesurée sous forme de métal.

6. Procédé selon la revendication 5,
**caractérisé en ce qu'**
on obtient des esters d'acide di(méth)acrylique qui présentent sans purification par distillation une teneur en zirconium inférieure à 0,1 ppm, mesurée sous forme de métal.

7. Procédé selon une ou plusieurs des revendications précédentes,
**caractérisé en ce qu'**
on utilise un ou plusieurs catalyseurs en une quantité de 0,01 à 10 % molaire, par rapport au nombre de moles de 1,n-diols.

8. Procédé selon une ou plusieurs des revendications précédentes,
**caractérisé en ce qu'**
on utilise un ou plusieurs catalyseurs en une quantité de 0,05 à 10 % molaire, par rapport au nombre de moles de 1,n-diols.

9. Procédé selon une ou plusieurs des revendications précédentes,
**caractérisé en ce qu'**
on conduit la trans-estérification en présence d'acétylacétonate de zirconium.

10. Procédé selon une ou plusieurs des revendications précédentes,
caractérisé en de qu'
on fait réagir du méthacrylate de méthyle ou de l'acrylate de méthyle avec des 1,n-diols, où n = 3, 4 ou 6.

11. Procédé selon une ou plusieurs des revendications précédentes,
**caractérisé en ce qu'**
on utilise de 2 à 20 moles d'acide (méth)acrylique pour une mole de 1,n-diol.

12. Procédé selon une ou plusieurs des revendications précédentes,
**caractérisé en ce qu'**
au cours de la réaction on retire de façon continue du mélange réactionnel l'alcanol de l'ester d'acide (méth)acrylique qui se libère.

13. Procédé selon une ou plusieurs des revendications précédentes,
**caractérisé en ce qu'**
on conduit la réaction en présence d'inhibiteurs de polymérisation.

14. Procédé selon une ou plusieurs des revendications précédentes,
**caractérisé en ce qu'** on conduit la réaction en présence de l'oxygène de l'air.
